# EUROPEAN PATENT APPLICATION

(11) **EP 4 095 147 A1**
(43) Date of publication of application: **30.11.2022**
(21) Application number: 20905858.5
(22) Date of filing: 23.12.2020
(51) Int. Cl.: C07K 1/02, C07K 1/06

(54) **METHOD FOR PRODUCING PEPTIDE**

(30) Priority: 23.12.2019 JP 2019231249
(71) Applicant: Otsuka Chemical Co., Ltd., Osaka-shi, Osaka 540-0021 (JP)
(72) Inventor: XU, Pengyu, Tokushima-shi, Tokushima 771-0193 (JP)
(74) Representative: Vossius & Partner Patentanwälte Rechtsanwälte mbB
(86) International application number: PCT/JP2020/048171
(87) International publication number: WO 2021/132336

(57) **Abstract**

A peptide is efficiently produced by reacting a carboxyl group of an amino acid or intermediate peptide in which an amino group is protected, with an alkali metal salt or alkaline earth metal salt of an amino acid in which an amino group is not protected, or of an intermediate peptide in which an N-terminal is not protected, in a two-layer solvent composed of water and a hydrophobic organic solvent.

## Description

### Technical Field

The present invention relates to a method for producing a peptide.

### Background Art

Peptides have long been studied in various fields due to their diversity and biocompatibility, and have a wide variety of applications, not only in pharmaceutical products but also in biochemical tools, food, and the like.

On the other hand, despite the fact that many synthetic methods for peptides have been studied over the years, a sufficiently satisfactory synthetic method has not yet been established. For example, solid phase peptide synthesis (SPPS), which is now becoming the standard peptide synthesis method, is expensive and has drawbacks for industrial-scale synthesis. Further, relatively inexpensive liquid-phase synthesis (LPPS) also has problems, such as its cumbersome protection and deprotection processes, easily occurring epimerisation, and complicated operation. In contrast, peptide-chain elongation reaction using unprotected amino acids in a microflow reactor has recently been reported (see, for example, Non-Patent Literature 1).

### Citation List

### Non-Patent Literature

NPL 1: Chem. Eur. J. 10.1002/chem.201903531

### Summary of Invention

### Technical Problem

However, in the method of NPL1, a purification process is required to perform the subsequent condensation reaction with a different amino acid because an unreacted raw material, side-reaction product, and the like are present in a reaction solution obtained after completion of the reaction.

An object of the present invention is to provide a method for efficiently producing a peptide.

### Solution to Problem

As a result of a variety of study, the present inventors found that by performing a reaction of condensing an unprotected amino acid in a two-layer solvent composed of water and a hydrophobic organic solvent, the subsequent amino acid condensation reaction can be performed without any special purification step. The inventors then conducted further research to accomplish the present invention.

Specifically, the present invention includes the peptide production methods shown in the following Items 1 to 4.

### Item 1

A method for producing a peptide, comprising
reacting a carboxyl group of an amino acid or intermediate peptide in which an amino group is protected,
with an alkali metal salt or alkaline earth metal salt of an amino acid in which an amino group is not protected, or of an intermediate peptide in which an N-terminal is not protected, in a two-layer solvent composed of water and a hydrophobic organic solvent.

### Item 2

The production method according to Item 1, further comprising reacting an alkali metal salt or alkaline earth metal salt of an identical of different amino acid in which an amino group is not protected, or of an identical or different intermediate peptide in which an N-terminal is not protected.

### Item 3

The production method according to Item 1 or 2, wherein the water is an aqueous solution exhibiting alkali.

### Item 4

The production method according to any one of Items 1 to 3, wherein the reaction is performed in the presence of a phase-transfer catalyst.

### Advantageous Effects of Invention

According to the production method of the present invention, peptide chain elongation reaction can be efficiently performed. More specifically, in successive reactions in which an amino acid is sequentially condensed with an amino acid or a terminal amino acid of a peptide, the subsequent reaction can be performed without requiring the step of isolating and purifying the peptide produced after completion of each reaction. Accordingly, the peptide production method of the present invention is extremely advantageous from the industrial viewpoint.

### Brief Description of Drawings

Fig. 1 shows an NMR chart of a peptide obtained in Example 1-1.
Fig. 2 shows an NMR chart of a peptide obtained in Example 1-2.
Fig. 3 shows an NMR chart of a protected peptide obtained in Example 2.
Fig. 4 shows an NMR chart of a peptide obtained in Example 2.

### Description of Embodiments

The present invention is detailed below.

In the present specification, the terms "comprise" and "contain" include comprising, consisting essentially of, and consisting of. In the present specification, the numerical range "A to B" indicates A or more to B or less unless otherwise specified.

In the present specification, the "(unprotected) amino group" includes not only -NH₂ but also a cyclic amino group in which a hydrogen atom on a nitrogen atom is not protected.

The peptide production method of the present invention is a production method comprising reacting a carboxyl group of an amino acid or intermediate peptide in which an amino group is protected, with an alkali metal salt or alkaline earth metal salt of an amino acid whose amino group is not protected, or of an intermediate peptide whose N-terminal is not protected, in a two-layer solvent composed of water and a hydrophobic organic solvent.

The amino acid or intermediate peptide in which an amino group is protected refers to an amino acid or intermediate peptide whose N-terminal is protected by a protecting group. The intermediate peptide in which an amino group is protected refers to an intermediate peptide in which not only the N-terminal amino group but also the amino group of a side chain is protected.

The production method of the present invention is not particularly limited and includes a method for producing a peptide represented by formula (1) (sometimes referred below to as "peptide of formula (1)"), comprising reacting an amino acid or intermediate peptide represented by the following formula (2) (sometimes referred below to as "amino acids of formula (2)") with an amino acid or intermediate peptide represented by formula (3) (sometimes referred below to as "amino acids of formula (3)") in a two-layer solvent composed of water and a hydrophobic organic solvent.

**Q-A¹-COOH** (2)

In the formula, A¹ represents an amino acid residue or peptide residue, and Q represents a protecting group of an amino group.

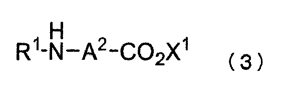

In the formula, A² represents an amino acid residue or peptide residue; R¹ represents a hydrogen atom or N-terminal substituent of amino acid, which taken together with A², may form a ring; and X¹ represents an alkali metal atom or alkaline earth metal atom.

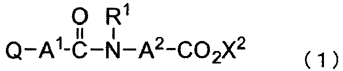

In the formula, Q, A¹, A², and R¹ are as defined above. X² represents a hydrogen atom, alkali metal atom, or alkaline earth metal atom.

The peptide production method of the present invention includes a production method comprising further reacting an alkali metal salt or alkaline earth metal salt of an amino acid whose amino group is not protected, or of an intermediate peptide whose N-terminal is not protected, with an intermediate peptide that is produced by reacting a carboxyl group of an amino acid or intermediate peptide in which an amino group is protected, with an alkali metal salt or alkaline earth metal salt of an amino acid whose amino group is not protected, or of an intermediate peptide whose N-terminal is not protected, in a two-layer solvent composed of water and a hydrophobic organic solvent. In this case, the amino acid whose amino group is not protected or the intermediate peptide whose N-terminal is not protected, which is reacted later, is selected according to the obtained peptide, and may be the same as or different from the amino acid whose amino group is not protected or the intermediate peptide whose N-terminal is not protected, which has been previously reacted.

Specifically, the production method of the present invention can be the method of producing a peptide represented by formula (5) (referred to below as "peptide of formula (5)"), comprising further reacting, in a two-layer solvent composed of water and a hydrophobic organic solvent, an amino acid or intermediate peptide represented by formula (4) (sometimes referred to below as "amino aides of formula (4)") with the peptide (X² = hydrogen atom) of formula (1) used as an intermediate peptide, wherein the peptide of formula (1) is produced by reacting the amino acids of formula (2) and the amino acids of formula (3) in the two-layer solvent composed of water and a hydrophobic organic solvent.

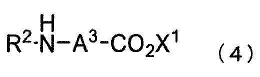

In the formula, X¹ is as defined above; A³ represents an amino acid residue or peptide residue; and R² represents a hydrogen atom or N-terminal substituent of amino acid, which taken together with A³, may form a ring.

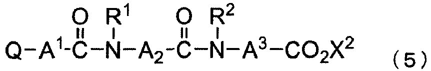

In the formula, Q, A¹, A², A³, R¹, R², and X² are as defined above.

After completion of the reaction, the produced peptide of formula (1) or peptide of formula (5) may be present in the reaction mixture in the form of an alkali metal salt or alkaline earth metal salt. Accordingly, when such an alkali metal salt or alkaline earth metal salt is removed as a peptide or used in the subsequent reaction, it may be prepared in the form of a carboxylic acid with an acid. The acid is not particularly limited as long as it can neutralize the alkali metal salt or alkaline earth metal salt of carboxylic acid, and examples include inorganic acids, such as hydrochloric acid, sulfuric acid, nitric acid, and phosphoric acid; and organic acids, such as formic acid, acetic acid, fumaric acid, and oxalic acid, with inorganic acids such as hydrochloric acid being preferred.

The amino acid constituting the amino acid or intermediate peptide in which an amino group is protected (amino acids of formula (2)), which is used in the production method of the present invention, is not particularly limited as long as it has amino and carboxyl groups in one molecule. Examples include glycine (Gly), alanine (Ala), valine (Val), leucine (Leu), isoleucine (Ile), phenylalanine (Phe), serine (Ser), threonine (Thr), lysine (Lys), 5-hydroxylysine (Hyl), arginine (Arg), aspartic acid (Asp), asparagine (Asn), glutamic acid (Glu), glutamine (Gln), cysteine (CySH), cystine (Cyss), cysteic acid (Cya), methionine (Met), tyrosine (Tyr), thyroxine (Thy), proline (Pro), hydroxyproline (Hyp), tryptophan (Trp), histidine (His), N-methylglycine, β-alanine, N-methyl-β-alanine, sarcosine, γ-aminobutyric acid, kainic acid, or derivatives thereof. Of these amino acids, preferable examples include glycine (Gly), alanine (Ala), valine (Val), leucine (Leu), isoleucine (Ile), phenylalanine (Phe), serine (Ser), threonine (Thr), lysine (Lys), 5-hydroxylysine (Hyl), arginine (Arg), aspartic acid (Asp), asparagine (Asn), glutamic acid (Glu), glutamine (Gln), cysteine (CySH), cystine (Cyss), cysteic acid (Cya), methionine (Met), tyrosine (Tyr), thyroxine (Thy), proline (Pro), hydroxyproline (Hyp), tryptophan (Trp), histidine (His), and the like. More preferable examples include glycine (Gly), valine (Val), leucine (Leu), isoleucine (Ile), phenylalanine (Phe), cysteic acid (Cya), methionine (Met), tyrosine (Tyr), thyroxine (Thy), proline (Pro), hydroxyproline (Hyp), tryptophan (Trp), and the like.

The peptide may be a compound in which two or more of the amino acids mentioned above are linked by a peptide bond, and the number of amino acids composing the peptide is usually 100 or less, preferably 50 or less, and more preferably 30 or less.

Examples of the protecting group for the amino group of the amino acid or peptide include amine protecting groups, such as benzyl (Bn), p-methoxybenzyl (PMB), and p-methoxyphenyl (PMP); amide protecting groups, such as formyl and acetyl (Ac); phthalimide protecting groups, such as phthaloyl (Phth); carbamate protecting groups, such as benzyloxycarbonyl (Cbz), tert-amyloxycarbonyl (Aoc), 9-fluorenylmethoxycarbonyl (Fmoc), tert-butyloxycarbonyl (Boc), allyloxycarbonyl (Alloc), and 2,2,2-triethoxycarbonyl (Troc); sulfonamide protecting groups, such as 3-nitro-2-pyridinesulphenyl (Npys), 2-nitrobenzenesulphonyl (Ns), and (2-trimethylsilyl)-ethanesulphonyl (SES); and the like. Of these, preferable examples include benzyloxycarbonyl (Cbz), 9-fluorenylmethoxycarbonyl (Fmoc), tert-butyloxycarbonyl (Boc), allyloxycarbonyl (Alloc), p-methoxybenzyl (PMB), phthaloyl (Phth), 2,2,2-triethoxycarbonyl (Troc), and the like. More preferable examples include benzyloxycarbonyl (Cbz), 9-fluorenylmethoxycarbonyl (Fmoc), tert-butyloxycarbonyl (Boc), p-methoxybenzyl (PMB), 2,2,2-triethoxycarbonyl (Troc), and the like.

The amino acid whose amino group is not protected or the intermediate peptide whose N-terminal is not protected (amino acids of formula (3) (or amino acids of formula (4)), which is to be reacted with the carboxyl group of the amino acid or intermediate peptide in which an amino group is protected, is not particularly limited as long as it has amino acid and carboxyl groups in one molecule. The amino acids that are listed as the amino acid composing the amino acid or intermediate peptide in which an amino group is protected can be selected and used according to the peptide to be produced.

In the production method of the present invention, the reaction is performed in a two-layer solvent of water and a hydrophobic organic solvent.

The hydrophobic organic solvent can be an organic solvent that is difficult to dissolve in water or mix with water. For example, the hydrophobic organic solvent is one that can form a clear boundary with the water layer after it is stirred with the same amount of water at 1 atm (1.013×10⁵ Pa) and 20°C, and allowed to stand. Specific examples include aliphatic hydrocarbons, such as n-hexane and n-heptane; halogenated aliphatic hydrocarbons, such as dichloromethane, chloroform, carbon tetrachloride, dichloroethane, trichloroethane, dichloropropane, and 1,2-dichloroethylene; aromatic hydrocarbons, such as benzene, toluene, xylene, and nitrobenzene; halogenated aromatic hydrocarbons, such as chlorobenzene, dichlorobenzene, and chlorotoluene; ester compounds, such as methyl acetate, ethyl acetate, butyl acetate, and ethyl formate; ether compounds, such as diethyl ether, cyclopentyl methyl ether (CPME), and tetrahydrofuran (THF); and the like.

Of these hydrophobic organic solvents, preferable examples include dichloromethane, chloroform, dichloroethane, trichloroethane, benzene, toluene, nitrobenzene, chlorobenzene, dichlorobenzene, chlorotoluene, methyl acetate, ethyl acetate, butyl acetate, ethyl formate, diethyl ether, cyclopentyl methyl ether, tetrahydrofuran, and the like; and more preferable examples include dichloromethane, chloroform, dichloroethane, trichloroethane, toluene, chlorobenzene, dichlorobenzene, chlorotoluene, diethyl ether, cyclopentyl methyl ether, and the like. This is because these are hydrophobic polar organic solvents and have alkali resistance and poor condensation reactivity.

These hydrophobic organic solvents can be used alone or in a mixture of two or more.

In the production method of the present invention, the amount of the hydrophobic organic solvent is not particularly limited and can be an amount in which the amino acid, intermediate peptide, or peptide in which an amino group is protected (amino acids of formula (2) or peptide of formula (1)) can be sufficiently dissolved. For example, the amount of the hydrophobic organic solvent is 0.1 to 100 parts by mass, preferably 1 to 50 parts by mass, and more preferably 2 to 10 parts by mass, per part by mass of the amino acid or peptide.

Water used as a solvent in the production method of the present invention may be tap water, ion-exchange water, distilled water, etc., as long as it does not adversely affect the production method of the present invention. An aqueous solution in which the amino acid whose amino group is not protected or the intermediate peptide whose N-terminal is not protected (amino acids of formula (3) (or amino acids of formula (4)) is dissolved is formed. Accordingly, the water is preferably an aqueous solution that exhibits alkali. The pH of the aqueous solution exhibiting alkali is greater than 7, preferably 7.1 or more, and more preferably 8 or more. The pH of the aqueous solution exhibiting alkali is preferably 14 or less, more preferably 11 or less, and even more preferably 10.5 or less.

In the production method of the present invention, the amino acids of formula (3) (or amino acids of formula (4)) are preferably used in the state of an aqueous solution. The amino acids of formula (3) (or amino acids of formula (4)) may be prepared by dissolving the carboxylic acid form of the amino acids of formula (3) (or amino acids of formula (4)) in an aqueous solution of an alkali metal salt, alkaline earth metal salt (alkali metal carbonates, alkaline earth metal carbonates, alkaline metal hydrogen carbonates, alkaline earth metal hydrogen carbonates, alkali metal hydroxides, alkaline earth metal hydroxides, etc.).

Examples of alkali metal carbonates include sodium carbonate and potassium carbonate, examples of alkaline earth metal carbonates include magnesium carbonate and calcium carbonate, examples of alkali metal hydrogen carbonates include sodium hydrogen carbonate and potassium hydrogen carbonate, examples of alkaline earth metal hydrogen carbonates include magnesium hydrogen carbonate and calcium hydrogen carbonate, examples of alkali metal hydroxides include sodium hydroxide and potassium hydroxide, and examples of alkaline earth metal hydroxides include magnesium hydroxide and calcium hydroxide.

The amount of water is not particularly limited, and it can be, for example, 0.5 to 100 parts by mass, preferably 1 to 50 parts by mass, and more preferably 2 to 10 parts by mass, per part by mass of the amino acid whose amino group is not protected or intermediate peptide whose N-terminal is not protected (amino acids of formula (3) (or amino acids of formula (4))).

The proportion of water and the hydrophobic organic solvent used in the production method of the present invention is not particularly limited, and the hydrophobic organic solvent is used in an amount of 0.05 to 20 parts by mass, preferably 0.1 to 10 parts by mass, and more preferably 0.33 to 3 parts by mass, per part by mass of water.

The production method of the present invention is a method for producing a peptide by subjecting an amino acid or intermediate peptide in which an amino group is protected (amino acids of formula (2) (or peptide of formula (1))), and an amino acid whose amino group is not protected or an intermediate peptide whose N-terminal is not protected (amino acids of formula (3) (or amino acids of formula (4)), to a condensation reaction. The reaction is preferably performed under such conditions that the amino acid whose amino group is not protected or the intermediate peptide whose N-terminal is not protected (amino acids of formula (3) (or amino acids of formula (4))) is dissolved in water.

On the other hand, it is preferable that the amino acid or intermediate peptide in which an amino group is protected (amino acids of formula (2) (or peptide of formula (1)) is dissolved in a hydrophobic organic solvent. In the reaction with the amino acid whose amino group is not protected or the intermediate peptide whose N-terminal is not protected, the carboxylic acid of the amino acid or intermediate peptide in which an amino group is protected (amino acids of formula (2) (or peptide of formula (1))) is preferably activated. As the activation means, it is possible to apply conventionally known condensation reactions for peptide bond formation, such as an azide method, mixed acid anhydride method, active ester method (e.g., chloroformic acid ester method, p-nitrophenyl ester method, and N-hydroxy succinic acid imide ester method), the Woodward reagent K method, a method using a condensing agent (e.g., dicyclohexylcarbodiimide (DCC), diisopropylcarbodiimide (DIC), 1-ethyl-3-(3-dimethylaminopropyl)carbodiimide hydrochloride (EDC), 2-chloro-1-methylpyridinium iodide (CMPI), benzotriazol-1-yl-oxy-tris(dimethylamino)phosphonium hexafluorophosphate (BOP), hexafluorophosphate(benzotriazole-1-yloxy)tripyrrolidinophosphonium (pyBOP), O-(7-azabenzotriazol-1-yl)-N,N,N,N-tetramethyluronium hexafluorophosphate (HATU), O-(benzotriazol-1-yl)-N,N,N,N-tetramethyluronium hexafluorophosphate (HBTU), (1-cyano-2-ethoxy-2-oxoethylideneaminooxy)dimethylaminomorpholinocarbenium hexafluorophosphate (COMU), etc.). Of these methods, the mixed acid anhydride method or active ester method can be particularly preferably applied.

With the substrate being present in each layer of the two-layer solvent as mentioned above, the solvent is stirred to advance the reaction, and the produced peptide (peptide of formula (1)) can be present in the hydrophobic organic solvent layer.

In this reaction, the proportion of the amino acid or intermediate peptide whose amino group is protected (amino acids of formula (2) (or peptide of formula (1))) to the amino acid whose amino group is not protected or the intermediate peptide whose N-terminal is not protected (amino acids of formula (3) (or amino acids of formula (4))) can be such that the amino acid whose amino group is not protected or the intermediate peptide whose N-terminal is not protected (amino acids of formula (3) (or amino acids of formula (4))) is 1 to 10 equivalents, preferably 1.1 to 5 equivalents, and more preferably 1.2 to 3 equivalents, relative to the amino acid or intermediate peptide whose amino group is protected (amino acids of formula (2) (or peptide of formula (1))).

The temperature of reaction is usually 4 to 80°C, preferably 10 to 50°C, and more preferably 20 to 30°C. The reaction time is usually about 0.1 to 100 hours, preferably 1 to 50 hours, and more preferably 5 to 20 hours. The temperature of reaction and reaction time can be determined according to the type and amount of the amino acid and peptide to be used.

In the production method of the present invention, a phase-transfer catalyst is preferably used to easily advance the reaction. The phase-transfer catalyst may be a catalyst known in organic synthetic chemistry. Examples include quaternary ammonium halides, such as tetramethylammonium bromide, tetramethylammonium chloride, tetraethylammonium bromide, tetrapropylammonium bromide, tetrabutylammonium bromide, tetrabutylammonium chloride, hexadecyltrimethylammonium bromide, benzyltriethylammonium chloride, and trioctylmethylammonium chloride; quaternary phosphonium halides, such as tetrabutylphosphonium bromide, benzyltriphenylphosphonium bromide, and butyltriphenylphosphonium bromide; crown ethers, such as 15-crown-5,18-crown-6, dibenzo-18-crown-6, dibenzo-24-crown-8, and dicyclohexyl-18-crown-6; and polyoxyalkylene glycols, such as polyethylene glycol, polypropylene glycol, and polyethylene glycol monomethyl ether; and the like.

The amount of the phase transfer catalyst is usually 0.0001 to 10 parts by mass, preferably 0.001 to 5 parts by mass, and more preferably 0.01 to 1 part by mass, per part by mass of the amino acid whose amino group is not protected or the intermediate peptide whose N-terminal is not protected (amino acids of formula (3) (or amino acids of formula (4)).

According to the production method of the present invention, the carboxyl group of the amino acid whose amino group is not protected or the intermediate peptide whose N-terminal is not protected (amino acids of formula (3)), which is used in the reaction does not need to be protected by a protecting group.

In particular, when the amino acid whose amino group is not protected is used, since the produced peptide (peptide of formula (1)) is present in the hydrophobic organic solvent, and the unreacted amino acid whose amino group is not protected (amino acid of formula (3)) is present in the water layer, the organic layer is collected by a liquid separation operation after completion of the reaction, which makes it possible to easily isolate the target peptide (peptide of formula (1)).

Furthermore, the obtained peptide (peptide of formula (1)) does not require a cumbersome deprotection reaction since no protecting group is present on the C-terminal carboxyl group. Even when the obtained peptide (peptide of formula (1)) is in the form of an alkali metal salt or alkaline earth metal salt, a peptide in the carboxyl form can be easily obtained by an acid treatment, as described above. For this reason, in order to further elongate the peptide chain, by applying the production method of the present invention, the obtained peptide (peptide of formula (1)) is optionally subjected to an acid treatment to form an intermediate peptide, and is subjected to condensation with the amino acid whose amino group is not protected or the intermediate peptide whose N-terminal is not protected (amino acids of formula (4)).

Specifically, according to the production method of the present invention, the amino acid or intermediate peptide whose amino group is protected (amino acids of formula (2)) is reacted with the amino acid whose amino group is not protected or the intermediate peptide whose N-terminal is not protected (amino acids of formula (3)) to produce a peptide (peptide of formula (1)); the obtained peptide of formula (1) is optionally subjected to an acid treatment to form an intermediate peptide, and the reaction mixture is separated to remove an organic layer (without requiring deprotection reaction of the carboxyl group); and the organic layer is used to react a new amino acid whose amino group is not protected or an intermediate peptide whose N-terminal is not protected (amino acids of formula (4)) in the alkali aqueous solution (without requiring deprotection reaction of the carboxyl group), thus producing a new peptide (peptide of formula (5)); and the reaction mixture is separated to obtain an organic layer in which the peptide (peptide of formula (5)) is present. By repeating these operations, a planned peptide can be efficiently produced.

The N-terminal protecting group of the obtained peptide (peptide of formula (1) (or peptide of formula (5))) can be deprotected by a known method.

### Examples

The present invention is explained below in detail below by means of Examples; however, the present invention is not limited by the Examples.

The measurement instruments used in the Examples are as follows.
NMR: nuclear magnetic resonance instrument produced by Bruker Corporation, 500 MHz
Measurement conditions: Heavy solvent CDCl₃ was used.
Magnetic field strength: 11.7 T
Purity: LC/MS: ACQUITY QDa detector for UPLC section and ACQUITY QDa detector for MS section, produced by Nihon Waters K.K.
Column used: CHIRALPAK ZWIX (+) produced by Daicel Corporation
Solvent: formic acid aqueous solution + diethylamine methanol solution
Measurement temperature: 25°C

### Example 1

### (1-1) Production of Boc-Gly-Pro-Hyp-OH

As a starting material, 0.8633 g (5 mmol) of glycine (Boc-Gly-OH) in which the N-terminal was protected by a tert-butyloxycarbonyl group (Boc) and the C-terminal was not protected was dissolved in 20 mL of dichloromethane, and 1.0253 g (7.5 mmol) of isobutyl chloroformate (CICOOiBu) and 0.7592 g (7.5 mmol) of triethylamine were added thereto and stirred. 15 mL of an aqueous sodium hydrogen carbonate solution (pH: 9.5) containing 0.8642 g (7.5 mmol) of proline (Pro) was added to the solution, and 0.20 g (10 mol%) of hexadecyltrimethylammonium bromide (CTAB) was added thereto, followed by stirring at room temperature (20°C) for 15 hours. The obtained reaction mixture was separated to remove an organic layer, and the organic layer was washed with 20 mL of saturated saline solution and dried.

1.0266 g (7.5 mmol) of isobutyl chloroformate and 0.7632 g (7.5 mmol) of triethylamine were added to the obtained organic layer, followed by stirring at 0°C. 15 mL of an aqueous sodium hydrogen carbonate solution (pH: 9.5) containing 1.0075 g (7.5 mmol) of hydroxyproline (Hyp) was added to the solution, followed by stirring at room temperature (20°C) for 15 hours. After 20 mL of an 1N hydrochloric acid aqueous solution was added to the reaction mixture, the mixture was separated to remove an organic layer. The obtained organic layer was washed sequentially with 20 mL of a 1N hydrochloric acid aqueous solution and 20 mL of a saturated saline solution. The obtained organic layer was concentrated under reduced pressure and dried in a vacuum line to obtain 1.2733 g (total yield: 66%) of a target peptide (Boc-Gly-Pro-Hyp-OH).

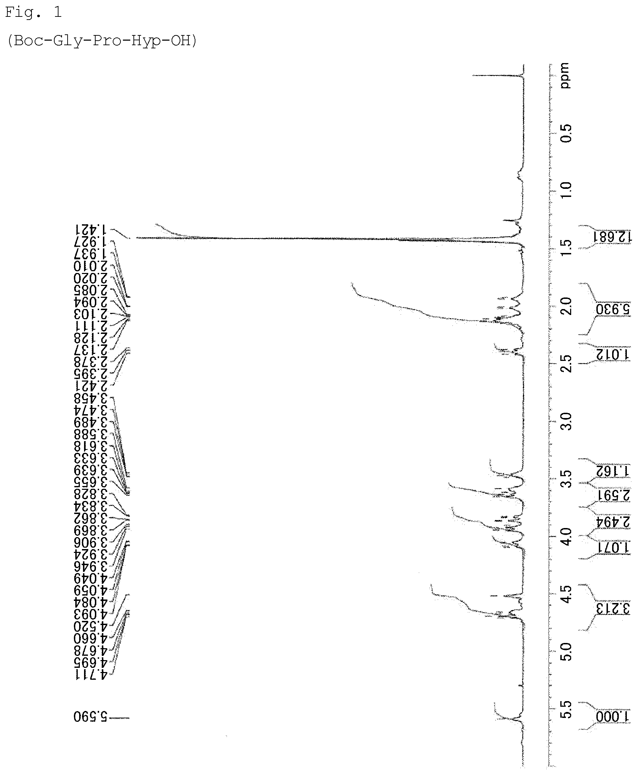

The NMR chart of the obtained peptide is shown in Fig. 1.

### (1-2) Production of Gly-Pro-Hyp-OH

After 50 mL of an ethyl acetate solution containing 4N hydrogen chloride was added at 0°C to the peptide obtained in Example 1-1 (Boc-Gly-Pro-Hyp-OH), stirring was performed until the temperature of the reaction system was returned to room temperature. Stirring was further performed for another 4 hours. The solid that had precipitated in the reaction mixture was filtered off and washed with 30 mL of ethyl acetate to obtain 1.0622 g (purity: 98%) of a target peptide (H-Gly-Pro-Hyp-OH hydrochloride).

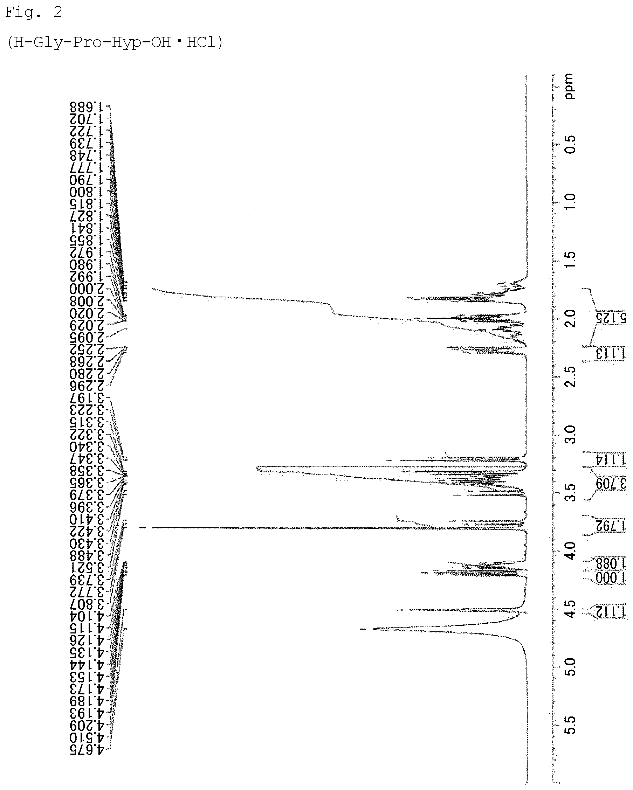

The NMR chart of the obtained peptide is shown in Fig. 2.

### Example 2 Production of Gly-Pro-Hyp-OH

As a starting material, 1.0420 g (5 mmol) of glycine (Cbz-Gly-OH) in which the N-terminal was protected by a benzyloxycarbonyl group (Cbz) and the C-terminal was not protected was dissolved in 20 mL of dichloromethane. 1.0300 g (7.5 mmol) of isobutyl chloroformate and 0.7599 g (7.5 mmol) of triethylamine were added thereto at 0°C, followed by stirring. 15 mL of an aqueous sodium hydrogen carbonate solution (pH: 9.5) containing 0.9001 g (7.5 mmol) of proline (Pro) was added to the solution, followed by the addition of 0.20 g (10 mol%) of hexadecyltrimethylammonium bromide (CTAB). Stirring was then performed at room temperature (20°C) for 15 hours. After 20 mL of a 1N hydrochloric acid aqueous solution was added to the obtained reaction mixture, the mixture was separated to remove an organic layer. The obtained organic layer was washed with 20 mL of a saturated saline solution and dried.

1.0097 g (7.5 mmol) of isobutyl chloroformate and 0.7625 g (7.5 mmol) of triethylamine were added to the obtained organic layer at 0°C, followed by stirring. 15 mL of an aqueous sodium hydrogen carbonate solution (pH: 9.5) containing 1.01 g (7.5 mmol) of hydroxyproline (Hyp) was added to this solution, followed by stirring at room temperature (20°C) for 15 hours. After 20 mL of a 1N hydrochloric acid aqueous solution was added to the reaction mixture, the mixture was separated to remove an organic layer. The obtained organic layer was washed sequentially with 20 mL of 1N hydrochloric acid and 20 mL of a saturated saline solution. The obtained organic layer was concentrated under reduced pressure and dried in a vacuum line to obtain 1.2916 g (total yield: 62%) of a protected peptide (CBz-Gly-Pro-Hyp-OH).

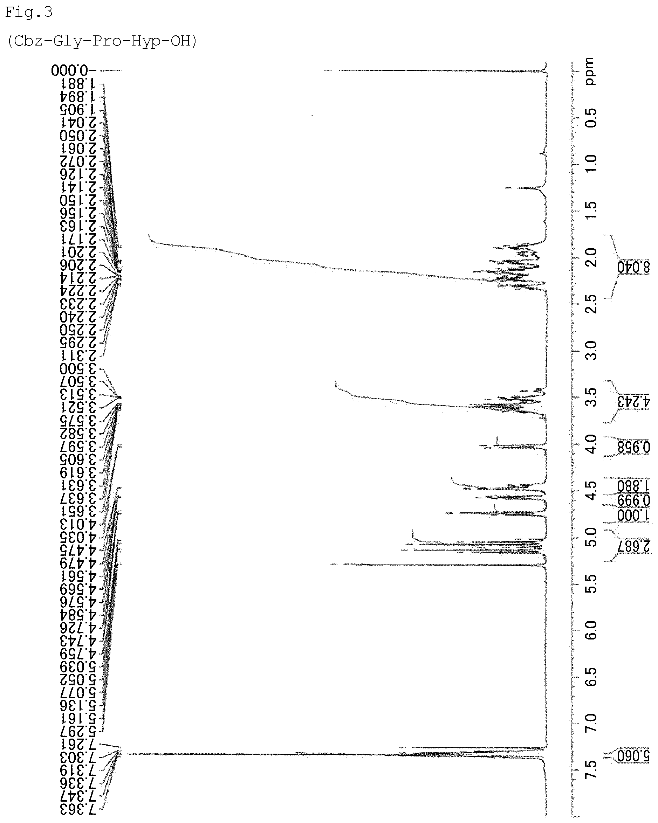

The NMR chart of the obtained protected peptide is shown in Fig. 3.

The protected peptide (Cbz-Gly-Pro-Hyp-OH) was subjected to, without any treatment, deprotection reaction for 54 hours under atmospheric pressure and hydrogen atmosphere by using 50 mg of a palladium carbon catalyst (Pd/C) and 10 mL of methanol, thus obtaining a target peptide (H-Gly-Pro-Hyp-OH).

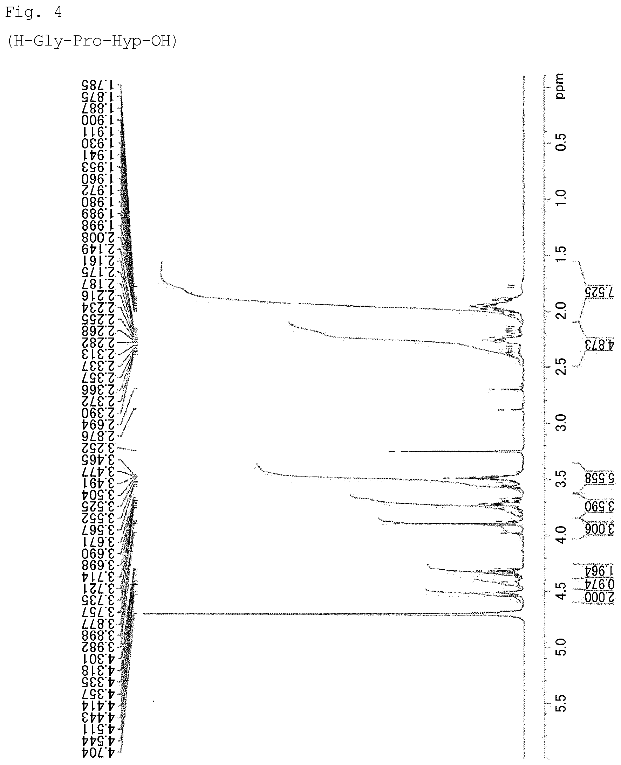

The NMR chart of the obtained peptide is shown in Fig. 4.

### Example 3 Synthesis of Boc-Gly-Pro-Gly-Pro-Gly-Val-Leu-OH

As a starting material, glycine (Boc-Gly-OH: 5 mmol) in which the N-terminal was protected by a tert-butyloxycarbonyl group (Boc) and the C-terminal was not protected was dissolved in 20 mL of dichloromethane, and 7.5 mmol of isobutyl chloroformate and 7.5 mmol of triethylamine were added thereto at 0°C, followed by stirring. 15 mL of an aqueous sodium hydrogen carbonate solution (pH: 9.5) containing 7.5 mmol of proline (Pro) was added to the solution, followed by the addition of 10 mol% of hexadecyltrimethylammonium bromide (CTAB). Stirring was then performed at room temperature (20°C) for 15 hours. After 20 mL of a 1N hydrochloric acid aqueous solution was added to the obtained reaction mixture, the mixture was separated to remove an organic layer. The obtained organic layer was washed with 20 mL of a saturated saline solution and dried.

7.5 mmol of isobutyl chloroformate and 7.5 mmol of triethylamine were added at 0°C to the obtained organic layer, followed by stirring. Thereafter, 15 mL of an aqueous sodium hydrogen carbonate solution (pH: 9.5) containing glycine (Gly: 7.5 mmol) was added thereto, followed by stirring at room temperature (20°C) for 15 hours. After 20 mL of a 1N hydrochloric acid aqueous solution was added to the obtained reaction mixture, the mixture was separated to remove an organic layer. The obtained organic layer was washed with 20 mL of a saturated saline solution and dried, thus obtaining an organic layer. After this, by repeating the above operation by changing the introduced amino acid to glycine, a target peptide can be produced. In this Example, the reaction was performed using proline in place of glycine, followed by sequential reactions with glycine, valine, and leucine, thus producing a target peptide (Boc-Gly-Pro-Gly-Pro-Gly-Val-Leu-OH).

The peptide formation was measured by LC-MS, and the MS peak, which was the same as that of the assumed compound, was confirmed.

## Claims

1. A method for producing a peptide, comprising
reacting a carboxyl group of an amino acid or intermediate peptide in which an amino group is protected,
with an alkali metal salt or alkaline earth metal salt of an amino acid in which an amino group is not protected, or of an intermediate peptide in which an N-terminal is not protected, in a two-layer solvent composed of water and a hydrophobic organic solvent.

2. The production method according to claim 1, further comprising reacting an alkali metal salt or alkaline earth metal salt of an identical of different amino acid in which an amino group is not protected, or of an identical or different intermediate peptide in which an N-terminal is not protected.

3. The production method according to claim 1 or 2, wherein the water is an aqueous solution exhibiting alkali.

4. The production method according to any one of claims 1 to 3, wherein the reaction is performed in the presence of a phase-transfer catalyst.
